# EUROPÄISCHE PATENTSCHRIFT

(11) **EP 1 874 314 B1**
(45) Veröffentlichungstag und Bekanntmachung des Hinweises auf die Patenterteilung: **18.08.2010**
(21) Anmeldenummer: 06722698.5
(22) Anmeldetag: 23.03.2006
(51) Int. Cl.: A61K 31/495, A61K 31/522, A61K 9/52

(54) **PELLETFÖRMIGE REATRDZUBEREITUNG ENTHALTEND CINNARIZIN UND DIMENHYDRINAT GEGEN SCHWINDEL**
PELLET-TYPE CONTROLLED-RELEASE PREPARATION CONTAINING CINNARIZINE AND DIMENHYDRINATE FOR COMBATING VERTIGO
PREPARATION A EFFET RETARD SOUS FORME DE GRANULES AGISSANT CONTRE LE VERTIGE

(30) Priorität: 23.03.2005 DE 102005014142
(43) Veröffentlichungstag der Anmeldung: 09.01.2008
(73) Patentinhaber: Hennig Arzneimittel GmbH & Co. KG, 65439 Flörsheim (DE)
(72) Erfinder: FRANCAS, Gernot, 67549 Worms (DE)
(74) Vertreter: Schubert, Klemens
(86) Internationale Anmeldenummer: PCT/DE2006/000546
(87) Internationale Veröffentlichungsnummer: WO 2006/099864

(56) Entgegenhaltungen:
- WO-A-2004/064843
- HALAMA P: "ERFAHRUNGEN BEI DER THERAPIE DES VESTIBULAEREN UND ZEREBRALEN SCHWINDELS MIT ARLEVERT THERAPIEKONTROLLE MITTELS KRANIOKORPOGRAPHIE TREATMENT OF VESTIBULAR AND CEREBRAL VERTIGO WITH CINNARIZINE PLUS DIMENHYDRINATE" THERAPIEWOCHE, KARLSRUHE, DE, Bd. 35, Nr. 12, 1985, Seiten 1422-1426, XP008036742 ISSN: 0040-5973
- "SCHWINDELTHERAPIE MIT CINNARIZIN UND DIMENHYDRINAT KOMBINATIONSBEHANDLUNG IST EFFEKTIVER THERAPY OF VERTIGO WITH CINNARIZINE AND DIMENHYDRINATE: COMBINATION TREATMENT IS MORE EFFECTIVE" TW NEUROLOGIE, PSYCHIATRIE, BRAUN, KARLSRUHE, DE, Bd. 11, Nr. 12, 1997, Seiten 927-928, XP008036746 ISSN: 0935-3224

## Beschreibung

Die Erfindung betrifft eine pharmazeutische Zusammensetzung einer Retardzubereitung in Form von Pellets zur Behandlung von Schwindel jedweder Genese.

Neben dem Kopfschmerz ist Schwindel (lat. vertigo) das häufigste von Patienten geklagte Symptom. Es handelt sich dabei um ein so genanntes multisensorisches Syndrom, das durch eine gestörte Wahrnehmung verschiedener Sinne gekennzeichnet ist und mit dem Verlust der Körpersicherheit im Raum und dadurch hervorgerufenen Gleichgewichtsstörungen einhergeht. In voller Ausprägung äußert sich Schwindel in der Wahrnehmung von Scheinbewegungen, in einer Fallneigung sowie in Übelkeit und Erbrechen. Schwindel kann sowohl episodisch als auch andauernd auftreten.

Unter Schwindel wird eine unangenehme Verzerrung der Raum- und Bewegungswahrnehmung verstanden, die zu Gleichgewichtsstörungen führt. Es handelt sich dabei nicht um eine eigenständige Krankheit, sondern um einen Symptomenkomplex unterschiedlicher Ursache und Herkunft, in den verschiedene Körpersinne involviert sind.

Aus der DE 103 01 981 A1 ist ein Präparat gegen Schwindel bekannt, welches Cinnarizin und Dimenhydrinat in Kombination enthält. Dabei wurde überraschenderweise gefunden, dass die Kombination der Wirkstoffe zu einem synergistischen Effekt führt.

Nachteilig ist hierbei jedoch, dass die Wirkung nach einiger Zeit nachlässt und es notwendig ist, mehrfach am Tage weitere Dosen zu applizieren. Bisher müssen diese Arzneimittel über den Tag verteilt bis zu 5 mal angewendet werden. Die damit einhergehenden Probleme in Bezug auf die Compliance sind somit klar.

Aufgabe der vorliegenden Erfindung ist es ein System zur Behandlung von Schwindel zur Verfügung zu stellen, dessen Wirkungsdauer gegenüber herkömmlichen Arzneimitteln verlängert ist.

Die Aufgabe der vorliegenden Erfindung wird durch eine pharmazeutische Zusammensetzung gemäß dem Hauptanspruch gelöst. Vorteilhafte Weiterbildungen der erfindungsgemäßen pharmazeutischen Zusammensetzung sind in den abhängigen Unteransprüchen gekennzeichnet.

Die Aufgabe wird erfindungsgemäß durch eine pharmazeutische Zusammensetzung enthaltend Cinnarizin und Dimenhydrinat gelöst, wobei die Wirkstofffreisetzung retardiert ist und die Zubereitung in Form von Pellets vorliegt, diese pharmazeutische Zusammensetzung weiterhin Bindemittel, Retardierungsmittel und Füllstoffe enthält, dass weitere Hilfsstoffe enthalten sind, wobei das Gewichtsverhältnis von Bindemittel / Füllstoff im Kern zwischen 50 / 1 und 5 / 1 sowie das Gewichtsverhältnis von Retardierungsmittel / weiteren Hilfsstoffen im Lack zwischen 4 / 1 und 1,5/ 1 liegt.

Ganz besonders bevorzugt ist eine erfindungsgemäße pharmazeutische Zusammensetzung, wobei das Gewichtsverhältnis von Bindemittel / Füllstoff im Kern zwischen 33,12 / 1 und 6,25 / 1 sowie das Gewichtsverhältnis von Retardierungsmittel / weiteren Hilfsstoffen im Lack zwischen 3 / 1 und 2,2 / 1 liegt.

Bevorzugt ist dabei ferner, dass das Bindemittel aus niedrigviskoser Hydroxypropylmethylcellulose (HPMC) ≤1000 cp, Mikrokristalliner Cellulose und/oder Polyethylenglykol (PEG) ausgewählt ist.

Bevorzugt ist dabei auch, dass das Retardierungsmittel aus niedrigviskoser Hydroxypropylmethylcellulose (HPMC) ≤1000 cp, und/oder Eudragit RL / RS / NE ausgewählt ist.

Besonders bevorzugt ist ferner, dass das Retardierungsmittel zugleich Filmbildner ist.

Ein weiterer Gegenstand der vorliegenden Erfindung ist die Verwendung der erfindungsgemäßen pharmazeutischen Zusammensetzung zur Behandlung von Schwindel jedweder Genese.

Ein weiterer Gegenstand der vorliegenden Erfindung ist auch die Verwendung *von Cinnarizin und Dimenhydrinat oder* deren physiologisch verträglichen Salzen in Kombination zur Herstellung einer Arzneiform zur Behandlung von Schwindel jedweder Genese.

Cinnarizin (CAS 293-57-7) ist der internationale Freiname (INN) für 1-Benzhydryl-4-trans-cinnamylpiperazin. Es handelt sich hierbei um ein Antiverginosum, das nach neuesten Erkenntnissen hauptsächlich als Kalziumkanalblocker an den vestibulären Haarzellen wirkt. Dimenhydrinat (CAS 523-87-5) ist der internationale Freiname (INN) für das 8-Chlortheophyllin-Salz des Diphenhydramins und ist ein Antihistaminikum mit anticholinergen Eigenschaften, das antivertiginös und antiemetisch wirkt. Die Löslichkeiten der Wirkstoffe in Wasser sind sehr unterschiedlich, nämlich Cinnarizin ca. 2 mg / 100 ml, Dimenhydrinat ca. 3 mg / ml.

Aufgrund des stark unterschiedlichen Löslichkeitsverhaltens ist es bisher nicht gelungen, eine Retardform zu schaffen, welche die Wirkstoffe in derart zeitnaher Weise freisetzt, dass der synergistische Effekt der Wirkstoffkombination erhalten bleibt.

Überraschenderweise wurde nun gefunden, dass eine Kombination bestimmter Hilfsstoffe, sofern diese in einem bestimmten Mengenverhältnis nebeneinander vorliegen, den erfindungsgemäßen retardierenden Effekt aufweisen.

Es konnte gezeigt werden, dass Bindemittel, Retardierungsmittel und Füllstoffe in einem bestimmten Verhältnis nebeneinander vorliegen müssen, um den erfinderischen Effekt der zeitnahen Freisetzung der Wirkstoffe zu bewirken.

Die Aufgabe der Erfindung wird durch eine pharmazeutische Zusammensetzung gelöst, welche im Hauptanspruch beschrieben ist. Vorteilhafte Ausgestaltungen der erfindungsgemäßen Zusammensetzung sind in den abhängigen Unteransprüchen gekennzeichnet.

Die Aufgabe wird somit durch die Schaffung einer retardierenden Zusammensetzung gelöst, welche Bindemittel, Retardierungsmittel und Füllstoffe in einem definierten Gewichtsverhältnis enthalten. Dieses erfindungsgemäße Gewichts-Verhältnis von Bindemittel : Füllstoff im Kern der Pellets liegt zwischen 50 : 1 und 5 : 1 sowie das Gewichtsverhältnis von Retardierungsmittel : weiteren Hilfsstoffen im Lack zwischen 4 : 1 und 1,5 : 1. Besonders bevorzugt ist dabei ein Gewichts-Verhältnis von Bindemittel : Füllstoff im Kern der Pellets zwischen 33,12 : 1 und 6,25 : 1 sowie das Gewichtsverhältnis von Retardierungsmittel : weiteren Hilfsstoffen im Lack zwischen 3 : 1 und 2,2 : 1.

Überraschender Weise wurde nämlich gefunden, dass in diesem Mengenverhältnis die Freisetzung der Wirkstoffe Dimenhydrinat und Cinnarizin im optimalen Verhältnis zueinander erfolgt. Nur so kann der synergistische Effekt der Kombination dieser Wirkstoffe erzielt werden.

Erfindungsgemäße pharmazeutische Zusammensetzung enthalten mindestens ein Bindemittel, welches aus niedrigviskoser Hydroxypropylmethylcellulose (HPMC) ≤ 1000 cp, Hydroxypropylcellulose (HPC), Mikrokristalliner Cellulose und/oder Polyethylenglykol (PEG) oder aus deren Mischungen ausgewählt ist. Allerdings können auch andere, dem Fachmann als gleichwertig bekannte Bindemittel erfindungsgemäß verwendet werden. Es ist auch möglich, die genannten und andere Bindemittel zu kombinieren, um zu erfindungsgemäßen Zusammensetzungen zu gelangen.

Erfindungsgemäße pharmazeutische Zusammensetzung enthalten ferner mindestens ein Retardierungsmittel, welches aus niedrigviskoser Hydroxypropylmethylcellulose (HPMC) ≤ 1000 cp, Eudragit RL, Eudragit RS und / oder Eudragit NE oder deren Mischungen ausgewählt ist. Allerdings können auch andere, dem Fachmann als gleichwertig bekannte Retardierungsmittel erfindungsgemäß verwendet werden. Es ist auch möglich, die genannten und andere Retardierungsmittel zu kombinieren, um zu erfindungsgemäßen Zusammensetzungen zu gelangen.

Erfindungsgemäße pharmazeutische Zusammensetzung enthalten ferner weitere Hilfsstoffe wie Weichmacher (z. B. Triacetin, PEG und andere), Trennmittel (z. B. Talkum, Glycerolmonostearat und andere), Farbstoffe und / oder Pigmente oder deren Mischungen ausgewählt ist. Derartige Hilfsstoffe sind beispielsweise Magnesiumstearat, Talkum, Aerosil, Trennmittel, Gleitmittel und dergleichen, welche die Verarbeitung der Mischungen mit Maschinen erleichtert und/oder ermöglicht. Allerdings können auch andere, dem Fachmann als gleichwertig bekannte weitere Hilfsstoffe erfindungsgemäß verwendet werden. Es ist auch möglich, die genannten und andere Hilfsstoffe zu kombinieren, um zu erfindungsgemäßen Zusammensetzungen zu gelangen.

Erfindungsgemäß geeignete Zubereitungsformen sind dem Fachmann bekannt und können Pellets, mit Pellets gefüllte Kapseln z.B. aus Gelatine und andere Formen sein.

Die Herstellung derartiger Arzneiformen ist dem Fachmann bekannt (e. g. Hagers Handbuch der Pharmazeut. Praxis, 5. Aufl. von 1990-1995 im Springer-Verlag, Berlin).

Die nachfolgenden Beispiele erläutern die Erfindung:

### Beispiel 1

Allgemeines Herstellungsverfahren zur Herstellung der erfindungsgemäßen Retardzubereitungen

Die Herstellung der erfindungsgemäßen pharmazeutischen Zusammensetzungen erfolgt in an sich bekannter Weise. Dabei werden
Dimenhydrinat, Cinnarizin, Bindemittel und Füllstoffe vorgemischt,
mit Hilfe von Wasser auf einer rotierenden Scheibe, in einer rotierenden Trommel oder im Extruder pelletiert, die so erhaltenen Pellets in den Korngrößenbereich 600 - 1200 µm klassiert,
dann die Pellets mit einer Suspension aus Retardierungsmittel und den weiteren Hilfsstoffen in Gegenwart von Alkohol lackiert
sowie die so erhaltenen lackierten Pellets in Gelatinekapseln gefüllt.

Es wurden erfindungsgemäße Zusammensetzungen nach verschiedenen Rezepturen wie oben angegeben hergestellt und deren Freisetzung nach der Paddle-Methode gemäß der Europäischen Pharmakopöe bestimmt.

Im folgenden wird die Erfindung anhand von Tabellen Figuren näher erläutert. Im Einzelnen zeigt
- Tabelle 2: eine tabellarische Darstellung der Freisetzung der erfindungsgemäßen pharmazeutischen Zusam- mensetzung gemäß Rezeptur 1 aus Tabelle 1
- Figur 1: ein Diagramm der Freisetzung von Cinnarizin,
- Figur 2: ein Diagramm der Freisetzung von Dimenhydrinat,
- Tabelle 3: eine tabellarische Darstellung der Freisetzung einer herkömmlichen Cinnarizin/Dimenhydrinat- Zubereitung,
- Figur 3: ein Diagramm der Freisetzung von Cinnarizin und
- Figur 4: ein Diagramm der Freisetzung von Dimenhydrinat.

Die Tabelle 2 zeigt eine tabellarische Darstellung der Freisetzung von Cinnariazin und Dimenhydrat bei Verwendung der erfindungsgemäßen pharmazeutischen Zusammensetzung gemäß Rezeptur 1 der Tabelle 1.

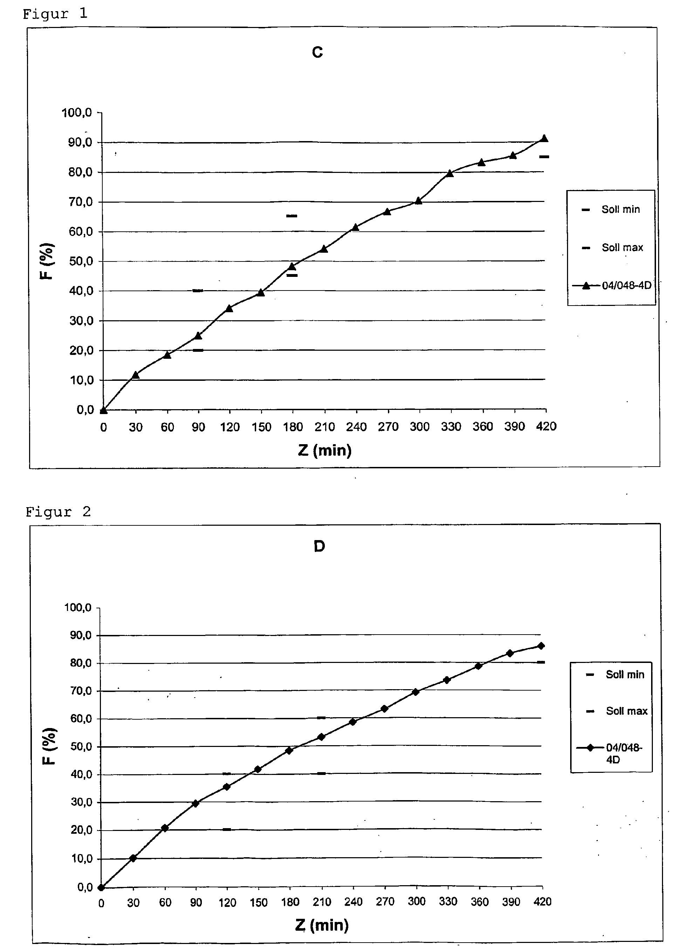

Ein Diagram der Freisetzung von Cinnarizin (W1) aus der erfindungsgemäßen Zusammensetzung ist in Figur 1 dargestellt.

Figur 2 zeigt ein Diagramm in dem die Freisetzung (F) von Dimenhydrinat (W2) in Abhängigkeit von der Zeit (Z) aufgetragen ist.

Im Vergleich zu Tabelle 2 zeigt die Tabelle 3 eine tabellarische Darstellung der Freisetzung von Cinnarizin und Dimenhydrat bei einer herkömmlichen, nicht erfindungsgemäßen Cinnariazin/Dimenhydrat-Zubereitung.

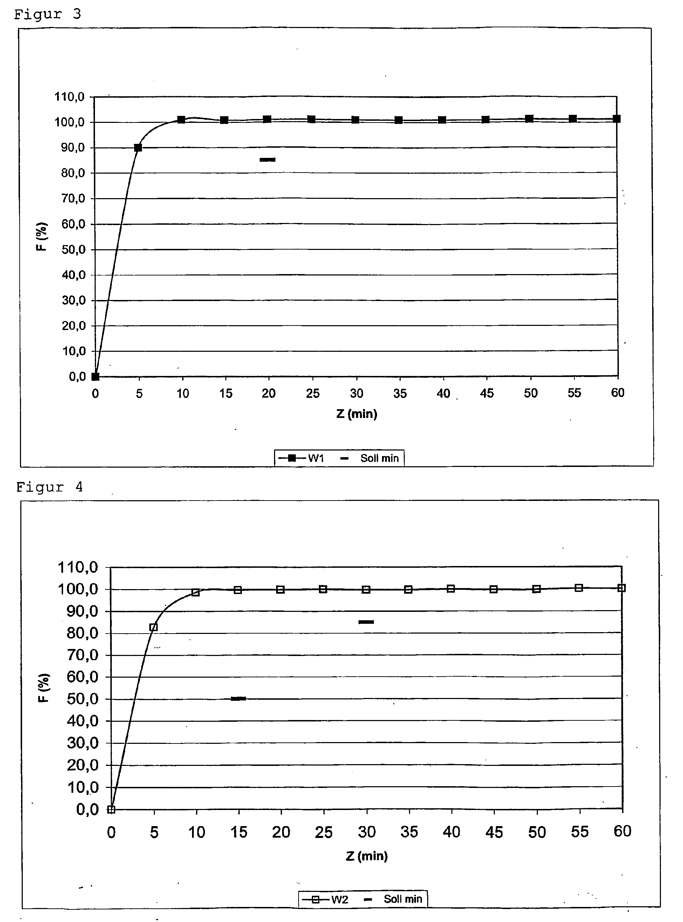

Die Figur 3 zeigt eine Diagramm in dem die Freisetzung (F) von Cinnarizin (W1) in Anhängigkeit von der Zeit (Z) aufgetragen ist.

Die Figur 4 zeigt ein Diagramm in dem die Freisetzung (F) von Dimenhydrinat (W2) in Abhängigkeit von der Zeit (Z) aufgetragen ist.

Aus der erfindungsgemäßen pharmazeutischen Zusammensetzung beträgt die In-vitro-Freisetzung der Cinnarizin Base nach 90 Minuten 20 % -40 %, nach 180 Minuten 45 % - 65 % und nach 420 Minuten > 85 %. Die In-vitro-Freisetzung von Dimenhydrinat beträgt nach 120 Minuten 20 % - 40 %, nach 210 Minuten 40 % - 60 % und nach 420 Minuten > 80 %.

Ein Vergleich der Figuren 1 und 3 sowie 2 und 4 zeigt, dass die Freisetzung von Cinnarizin bzw. Dimenhydrat bei der unretardierten Mischung von Cinnarizin und Dimenhydrat zunächst stark ansteigt und dann auf einem Wert von ca. 100 % bleibt, während der Anstieg der Freisetzung bei der erfindungsgemäßen Zusammensetzung verzögert stattfindet.

### Beispiele 2-6

Die folgende Tabelle 1 zeigt Rezepturen zur Herstellung von erfindungsgemäßen Zubreitungen in Form von Pellets.

**Tabelle 1**

| | **Rezepturen** | | | | |
|---|---|---|---|---|---|
| **Bestandteil** | **1** | **2** | **3** | **4** | **5** |
| **Wirkstoff** | | | | | |
| Dimenhydrinat | 120 | 120 | 120 | 120 | 120 |
| Cinnarizin | 60 | 60 | 60 | 60 | 60 |

| **Bindemittel** | | | | | |
|---|---|---|---|---|---|
| Mikrokristalline | | | | | |
| Cellulose | 100 | 110 | 132 | 100 | 110 |
| HPMC niedrigviskos | 20 | 20 | 7 | 20 | 20 |
| PEG | 5 | 5 | 1,75 | 5 | 5 |

| **Füllstoff** | | | | | |
|---|---|---|---|---|---|
| Maisstärke | 20 | 10 | 4,25 | 20 | 10 |
| **Pellet - Menge** | 325 mg | | | | |
| **Pellet- Größe** | 600 - 1200 µm | | | | |

| **Retardierungsmittel = Filmbildner** | | | | | |
|---|---|---|---|---|---|
| Eudragit RS | 10 | | | 10 | 10 |
| Eudragit RL | | | 10 | | |
| Eudragit NE | | 10 | | | |
| HPMC niedrigviskos | 2 | 2 | 1 | 1 | 2 |

| **Weichmacher** | | | | | |
|---|---|---|---|---|---|
| Triacetin | 2 | | 2 | 2 | 2 |
| PEG | | | | 1 | |

| **Trennmittel** | | | | | |
|---|---|---|---|---|---|
| Talkum | 2 | 4 | | 2 | 1 |
| Glycerolmonostearat | | | 3,7 | | |

| **Farbstoffe** | | | | | |
|---|---|---|---|---|---|
| FeOx | | | 0,1 | | 0,5 |

| **Pigmente** | | | | | |
|---|---|---|---|---|---|
| Titandioxid | | | 0,2 | | 0,5 |
| **Film- Menge** | 16 mg | 16 mg | 16 mg | 16 mg | 16 mg |
| **Retardpellet- Menge** | 341 mg | | | | |

Auch diese erfindungsgemäßen Rezepturen weisen die erfinderischen Vorteile auf, indem die Wirkstoffe in zeitnaher Weise freigesetzt werden, so dass deren synergistischer Effekt erhalten bleibt.

**Tabelle 2**

| **Zeit (min)** | Freisetzungs- Mittelwerte (%) | | | | | |
|---|---|---|---|---|---|---|
| | Cinnarizin (C) | | | Dimenhydrinat (D) | | |
| | **Soll min** | **Soll max** | **04/048-4D** | **Soll min** | **Soll max** | **04/048-4D** |
| **0** | | | 0,0 | | | 0,0 |
| **30** | | | 11,8 | | | 10,2 |
| **60** | | | 18,5 | | | 20,7 |
| **90** | **20,0** | **40,0** | 25,0 | | | 29,4 |
| **120** | | | 34,2 | **20,0** | **40,0** | 35,4 |
| **150** | | | 39,5 | | | 41,8 |
| **180** | **45,0** | **65,0** | 48,1 | | | 48,5 |
| **210** | | | 54,1 | **40,0** | **60,0** | 53,2 |
| **240** | | | 61,5 | | | 58,5 |
| **270** | | | 66,8 | | | 63,2 |
| **300** | | | 70,5 | | | 69,2 |
| **330** | | | 79,6 | | | 73,6 |
| **360** | | | 83,3 | | | 78,6 |
| **390** | | | 85,6 | | | 83,2 |
| **420** | **85,0** | | 91,2 | **80,0** | | 85,9 |

**Tabelle 3**

| **Zeit (Z) (min)** | Freisetzungs- Mittelwerte (%)(F) | | | |
|---|---|---|---|---|
| | Cinnarizin (W1) | | Dimenhydrinat (W2) | |
| | **Soll min** | **03/633** | **Soll min** | **03/633** |
| **0** | | 0,0 | | 0,0 |
| **5** | | 89,9 | | 82,8 |
| **10** | | 100,9 | | 98,5 |
| **15** | | 100,8 | **50,0** | 99,5 |
| **20** | **85,0** | 100,9 | | 99,8 |
| **25** | | 101,0 | | 100,0 |
| **30** | | 100,9 | **85,0** | 99,7 |
| **35** | | 100,9 | | 99,8 |
| **40** | | 101,0 | | 100,0 |
| **45** | | 100,9 | | 99,7 |
| **50** | | 101,1 | | 99,9 |
| **55** | | 101,3 | | 100,3 |
| **60** | | 101,2 | | 100,1 |

## Patentansprüche

1. Pharmazeutische Zusammensetzung enthaltend Cinnarizin und Dimenhydrinat, **dadurch gekennzeichnet, dass** die Wirkstofffreisetzung retardiert ist und die Zubereitung in Form von Pellets vorliegt,
dass diese weiterhin Bindemittel, Retardierungsmittel und Füllstoffe enthält,
dass weitere Hilfsstoffe enthalten sind, und
dass das Gewichtsverhältnis von Bindemittel / Füllstoff im Kern zwischen 50 / 1 und 5 / 1 sowie das Gewichtsverhältnis von Retardierungsmittel / weiteren Hilfsstoffen im Lack zwischen 4 / 1 und 1,5/ 1 liegt.

2. Pharmazeutische Zusammensetzung gemäß Anspruch 1, **dadurch gekennzeichnet, dass** das Gewichtsverhältnis von Bindemittel / Füllstoff im Kern zwischen 33,12 / 1 und 6,25 / 1 sowie das Gewichtsverhältnis von Retardierungsmittel / weiteren Hilfsstoffen im Lack zwischen 3 / 1 und 2,2 / 1 liegt.

3. Pharmazeutische Zusammensetzung gemäß einem der voranstehenden Ansprüche, **dadurch gekennzeichnet, dass** das Bindemittel aus niedrigviskoser Hydroxypropylmethylcellulose (HPMC) ≤ 1000 cp, Mikrokristalliner Cellulose und/oder Polyethylenglykol (PEG) ausgewählt ist.

4. Pharmazeutische Zusammensetzung gemäß einem der voranstehenden Ansprüche, **dadurch gekennzeichnet, dass** das Retardierungsmittel aus niedrigviskoser Hydroxypropylmethylcellulose (HPMC) ≤ 1000 cp, und/oder Eudragit RL / RS / NE ausgewählt ist.

5. Pharmazeutische Zusammensetzung gemäß einem der voranstehenden Ansprüche, **dadurch gekennzeichnet, dass** das Retardierungsmittel zugleich Filmbildner ist.

6. Verwendung der pharmazeutischen Zusammensetzung nach einem der voranstehenden Ansprüche zur Herstellung einer Arzneiform zur Behandlung von Schwindel jedweder Genese.

## Claims

1. A pharmaceutical composition containing cinnarizine and dimenhydrinate, hereby **characterized in that** the release of active ingredients is slowed down and the preparation is present in the form of pellets, and that it additionally contains binding agent, slow-release agent and fillers, and that additional auxiliary agents are contained in it, and that the weight ratio of binding agent/filler in the core lies between 50 / 1 and 5 / 1 and the weight ratio of slow-release agent/additional auxiliary agents in the lacquer lies between 4 / 1 and 1.5 / 1.

2. The pharmaceutical composition according to claim 1, **characterized in that** the weight ratio of binding agent/filler in the core lies between 33.12 / 1 and 6.25 / 1 and the weight ratio of slow-release agent/additional auxiliary agents in the lacquer lies between 3 / 1 and 2.2 / 1.

3. The pharmaceutical composition according to one of the preceding claims, **characterized in that** the binding agent is selected from low-viscosity hydroxypropylmethylcellulose (HPMC) ≤ 1000 cp, microcrystalline cellulose and/or polyethylene glycol (PEG).

4. The pharmaceutical composition according to one of the preceding claims, **characterized in that** the slow-release agent is selected from low-viscosity hydroxypropylmethylcellulose (HPMC) ≤ 1000 cp and/or Eudragit RL / RS / NE.

5. The pharmaceutical composition according to one of the preceding claims, **characterized in that** the slow-release agent is also a film forming agent.

6. The use of the pharmaceutical composition according to one of the preceding claims for the production of a medicament for the treatment of vertigo of any genesis.

## Revendications

1. Composition pharmaceutique contenant de la cinnarizine et du diménhydrinate, **caractérisée en ce que** la libération de la substance active est retardée et la préparation est présente sous la forme de granulés ; **en ce qu'**elle contient en outre des liants, des agents de retardement et des substances de charge ; **en ce qu'**elle contient des adjuvants supplémentaires ; et **en ce que** le rapport pondéral du liant / substance de charge dans le noyau se situe entre 50 / 1 et 5 / 1 et le rapport pondéral de l'agent de retardement / substances auxiliaires supplémentaires dans la gomme laque se situe entre 4/1 et 1,5 / 1.

2. Composition pharmaceutique selon la revendication 1, **caractérisée en ce que** le rapport pondéral du liant / substance de charge dans le noyau se situe entre 33,12 / 1 et 6,25 / 1 et le rapport pondéral de l'agent de retardement / substances auxiliaires supplémentaires dans la gomme laque se situe entre 3 / 1 et 2,2 / 1.

3. Composition pharmaceutique selon l'une quelconque des revendications précédentes, **caractérisée en ce que** le liant est choisi parmi de l'hydroxypropylméthylcellulose (HPMC) à faible viscosité ≤ 1000 cp, de la cellulose microcristalline et/ou du polyéthylèneglycol (PEG).

4. Composition pharmaceutique selon l'une quelconque des revendications précédentes, **caractérisée en ce que** l'agent de retardement est choisi parmi de l'hydroxypropylméthylcellulose (HPMC) faible viscosité ≤ 1000 cp, et/ou de l'Eudragit RL / ES / NE.

5. Composition pharmaceutique selon l'une quelconque des revendications précédentes, **caractérisée en ce que** l'agent de retardement fait également office d'agent filmogène.

6. Utilisation de la composition pharmaceutique selon l'une quelconque des revendications précédentes pour la préparation d'une forme médicamenteuse destinée au traitement du vertige quelle que soit son origine.
